# FASCICULE DE BREVET EUROPEEN

(11) **EP 0 551 680 B1**
(45) Date de publication et mention de la délivrance du brevet: **15.11.1995**
(21) Numéro de dépôt: 92204108.2
(22) Date de dépôt: 29.12.1992
(51) Int. Cl.: C07C 17/38, C07C 19/08

(54) **Procédé pour l'épuration de 1,1-dichloro-1-fluoroéthane**
Verfahren zur Reinigung von 1,1-Dichlor-1-Fluorethan
Process for the purification 1,1-dichloro-1-fluorethane

(30) Priorité: 13.01.1992 BE 9200026
(43) Date de publication de la demande: 21.07.1993
(73) Titulaire: SOLVAY (Société Anonyme), B-1050 Bruxelles (BE)
(72) Inventeur: Lechaude, Paul, B-1050 Bruxelles (BE); Darago, Gilles, F-39500 Tavaux (FR); Krafft, Philippe, F-39500 Tavaux (FR); Catinat, Jean-Pierre, B-7130 Binche (BE)
(74) Mandataire: Jacques, Philippe

(56) Documents cités:
- EP-A- 0 420 709

## Description

La présente invention concerne un procédé perfectionné pour l'épuration de 1,1-dichloro-1-fluoroéthane par traitement avec du chlore suivi de distillation.

Le 1,1-dichloro-1-fluoroéthane (HFA-141b) est un hydrocarbure chlorofluoré partiellement halogéné qui s'avère être un substitut intéressant de certains chlorofluorocarbures entièrement halogénés (CFC) dont la production et l'utilisation sont progressivement réduites parce qu'ils sont suspectés d'avoir un effet néfaste sur la couche d'ozone.

Les compositions issues des procédés pour la préparation du 1,1-dichloro-1-fluoroéthane comprennent des sous-produits et des impuretés indésirables. Certains de ces sous-produits et impuretés peuvent être aisément séparés par distillation. C'est notamment le cas pour le 1-chloro-1,1-difluoroéthane et le 1,1,1-trifluoroéthane, ainsi que pour les oligomères formés en cours de réaction. Toutefois, le 1,1-dichloro-1-fluoroéthane contient généralement en tant qu'impuretés de petites quantités de composés insaturés chlorés et/ou chlorofluorés dont la séparation par distillation s'avère difficile, vu leur point d'ébullition voisin de celui du 1,1-dichloro-1-fluoroéthane.

Outre le chlorure de vinylidène, qui constitue l'impureté la plus importante, les impuretés insaturées qui peuvent être présentes dans le 1,1-dichloro-1-fluoroéthane à purifier sont principalement les 1,2-dichloro-1-fluoroéthylènes cis et trans, le 1,2-dichloroéthylène trans ainsi que des traces de dichloroacétylène et de 1-chloro-1-fluoroéthylène.

La demande de brevet EP-A-0420709 de ATOCHEM décrit la purification du 1,1-dichloro-1-fluoroéthane essentiellement en chlorure de vinylidène et en dichloroacétylène par action du chlore et/ou d'un hydracide, avec un rapport molaire entre d'une part le chlore et/ou l'hydracide et d'autre part le chlorure de vinylidène au moins égal à 1,2 et pouvant aller jusque 10, en présence de 0,001 à 1 % en poids d'acide de Lewis par rapport au produit brut à traiter, suivie d'une séparation par distillation. La majorité des exemples décrivent la chloration des impuretés sous l'action du chlore en l'absence d'hydracide. L'exemple 9 décrit la chloration en présence de SbCl₅ d'un soutirage d'une fabrication de 1,1-dichloro-1-fluoroéthane et de 1-chloro-1,1-difluoroéthane contenant 2 % en poids d'hydracide (HCl plus HF) pendant 3 heures à 9°C pour obtenir un produit épuré contenant moins de 70 ppm de chlorure de vinylidène.

Ce procédé de l'art antérieur ne permet dès lors pas une épuration rapide en chlorure de vinylidène en présence d'un acide de Lewis et de fluorure d'hydrogène. De plus, l'élimination des 1,2-dichloro-1-fluoroéthylènes cis et trans apparait difficile car il a été observé par ailleurs que, dans certains cas, après être passée par un minimum, leur concentration croit si le traitement de chloration est prolongé. Enfin, la mise en oeuvre d'acide de Lewis dans des quantités telles que préconisées dans cette demande peut avoir pour conséquence une importante dégradation du 1,1-dichloro-1-fluoroéthane en aval de l'étape de chloration.

La présente invention a dès lors pour but de procurer un procédé perfectionné pour l'épuration de 1,1-dichloro-1-fluoroéthane brut par traitement avec du chlore des impuretés insaturées tant chlorées que chlorofluorées pour former des composés saturés aisément séparables par distillation qui soit rapide et ne provoque pas de dégradation significative du 1,1-dichloro-1-fluoroéthane.

A cet effet, l'invention concerne un procédé pour l'épuration de 1,1-dichloro-1-fluoroéthane brut par traitement avec du chlore en présence d'acide de Lewis et de fluorure d'hydrogène puis distillation, le fluorure d'hydrogène étant présent lors du traitement avec du chlore à raison d'au moins 5 % en poids par rapport au mélange de 1,1-dichloro-1-fluoroéthane brut et de fluorure d'hydrogène.

Un effet surprenant de la présente invention réside dans le fait que la mise en oeuvre de l'étape de traitement avec du chlore en présence de faibles quantités de fluorure d'hydrogène offre de moins bons résultats qu'en l'absence de fluorure d'hydrogène, tandis qu'en présence d'au moins 5 % en poids de fluorure d'hydrogène par rapport au mélange de 1,1-dichloro-1-fluoroéthane brut et de fluorure d'hydrogène, la chloration des impuretés insaturées est nettement plus rapide.

Le plus souvent, le traitement avec du chlore est réalisé en présence d'au moins 15 % en poids de fluorure d'hydrogène. La quantité maximale de fluorure d'hydrogène mis en oeuvre lors du traitement avec le chlore n'est pas véritablement critique. Néanmoins, en vue de limiter la dégradation de 1,1-dichloro-1-fluoroéthane en 1-chloro-1,1-difluoroéthane et l'excès de fluorure d'hydrogène à recycler en aval du traitement d'épuration, il est préférable de ne pas dépasser 50 % en poids de fluorure d'hydrogène par rapport au mélange de 1,1-dichloro-1-fluoroéthane brut et de fluorure d'hydrogène.

En pratique, le fluorure d'hydrogène nécessaire peut provenir en tout ou partie de l'excès initial de fluorure d'hydrogène mis en oeuvre pour la préparation du 1,1-dichloro-1-fluoroéthane et dès lors présent dans le 1,1-dichloro-1-fluoroéthane brut à traiter. L'étape de traitement avec du chlore peut ainsi être réalisée sans séparation préalable du fluorure d'hydrogène n'ayant pas réagi et éventuellement sans ajouter de fluorure d'hydrogène pour la seule étape de chloration des impuretés insaturées comprises dans le 1,1-dichloro-1-fluoroéthane brut.

Par ailleurs, la quantité d'acide de Lewis mis en oeuvre est avantageusement inférieure à 10 ppm par rapport au 1,1-dichloro-1-fluoroéthane brut à traiter, pour éviter une dégradation excessive du 1,1-dichloro-1-fluoroéthane en aval de la présente étape, notamment dans les étapes suivantes de distillation. La quantité d'acide de Lewis mis en oeuvre peut être aussi faible que 0,15 ppm. Bien que cette quantité soit très faible, la présence d'un acide de Lewis reste indispensable pour réaliser la chloration de toutes les impuretés insaturées. On utilise de préférence au moins 1,5 ppm d'acide de Lewis.

Un autre aspect surprenant de l'invention réside dans le fait que la mise en oeuvre de quantités aussi faibles d'acide de Lewis s'avère très efficace en vue de l'épuration du 1,1-dichloro-1-fluoroéthane en impuretés insaturées. Vu les très faibles quantités d'acide de Lewis utilisées, le procédé selon l'invention présente en outre l'avantage de rendre superflue une étape d'élimination de l'acide de Lewis par lavage par une solution aqueuse acide ou par complexation, préalablement à toute étape ultérieure, alors qu'une telle étape est nécessaire afin d'éviter la dégradation du 1,1-dichloro-1-fluoroéthane et la réapparition d'impuretés insaturées lorsque le traitement avec du chlore est réalisé en présence de quantités plus importantes d'acide de Lewis.

Généralement, l'acide de Lewis est choisi parmi FeCl₃, SnCl₄, SbCl₅, MoCl₅, TiCl₄ et leurs mélanges. On utilise de préférence FeCl₃.

L'acide de Lewis peut être introduit sous différentes formes et de différentes manières. Il peut notamment être introduit par dissolution dans une fraction du flux de 1,1-dichloro-1-fluoroéthane brut ou sous la forme d'une solution dans d'autres solvants tels notamment le tétrachlorométhane, le trichlorométhane ou le 1,2-dichloroéthane.

La très faible quantité d'acide de Lewis nécessaire selon l'invention peut en tout ou partie être formée à partir du métal provenant de l'installation, éventuellement sans nécessiter d'apport extérieur. En particulier, FeCl₃ peut être généré in situ à partir de fer provenant de l'installation. Il peut notamment en être ainsi lorsque l'installation comporte au moins une boucle de recyclage, par exemple du fluorure d'hydrogène. Dans ce cas, il convient de contrôler la quantité de fer qui circule dans l'installation afin d'éviter qu'elle ne dépasse la quantité admise selon l'invention et n'ait pour conséquence les inconvénients déjà évoqués.

Le chlore est introduit en quantités excédentaires par rapport aux impuretés insaturées à chlorer. Généralement, le traitement avec du chlore est réalisé en présence d'au moins 0,1 % en poids de chlore par rapport au poids du 1,1-dichloro-1-fluoroéthane brut à traiter. Le plus souvent, on ne dépasse pas 12 % de chlore. De préférence, on utilise au moins 1,5 % en poids de chlore. Avantageusement, on préfère ne pas mettre en oeuvre plus de 8 % en poids de chlore par rapport au poids du 1,1-dichloro-1-fluoroéthane brut à traiter.

Le traitement avec du chlore peut être réalisé à une température généralement d'au moins 0°C. Pour obtenir une bonne vitesse de chloration, on préfère travailler à une température d'au moins 60°C. Pour éviter une dégradation importante du 1,1-dichloro-1-fluoroéthane en 1,1,2-trichloro-1-fluoroéthane et la reformation de 1,2-dichloro-1-fluoroéthylènes cis et trans, il est particulièrement préférable de ne pas dépasser une température de 90°C.

Le traitement avec du chlore peut être effectué à pression atmosphérique ou à une pression supérieure à la pression atmosphérique. Cette pression peut être la pression autogène ou une pression supérieure générée par l'introduction d'un gaz inerte tel que l'hélium.

Dans les conditions opératoires retenues selon l'invention, une chloration quasi complète des impuretés insaturées chlorées et chlorofluorées peut être réalisée avec un temps de traitement de 1 à 60 minutes. De préférence, le traitement avec du chlore dure au moins 2 minutes. Pour limiter la dégradation du 1,1-dichloro-1-fluoroéthane et la reformation de 1,2-dichloro-1-fluoroéthylènes cis et trans, on préfère ne pas dépasser 30 minutes. On observe d'excellents résultats avec un temps de traitement d'environ 15 minutes.

Afin notamment d'éviter de générer dans le milieu des quantités excessives de FeCl₃, le réacteur de chloration et les appareillages en aval sont de préférence réalisés avec des matériaux résistant à la corrosion tels que notamment les alliages MONEL, INCONEL, HASTELLOY.

Après le traitement avec du chlore, on obtient du 1,1-dichloro-1-fluoroéthane de grande pureté en éliminant par distillation les composés inorganiques, essentiellement le fluorure d'hydrogène et le chlore n'ayant pas réagi, qui peuvent éventuellement être recyclés, et les composés organiques plus légers et plus lourds que le 1,1-dichloro-1-fluoroéthane, initialement présents ou formés durant le traitement au chlore.

L'exemple 1 est donné dans le but d'illustrer l'invention mais il n'est nullement limitatif. Les exemples 2 et 3 sont donnés à titre de référence.

### Exemples

### Exemple 1

Dans un autoclave en alliage HASTELLOY de 0,5 litre, équipé d'un agitateur, préalablement refroidi à -35°C et mis sous vide de 1500 Pa, on introduit par aspiration 253 g de 1,1-dichloro-1-fluoroéthane à épurer contenant 3,8 ppm de FeCl₃. On y ajoute 120 g de fluorure d'hydrogène, préalablement maintenu à température ambiante dans un cylindre en acier inoxydable. Le mélange de 1,1-dichloro-1-fluoroéthane brut et de fluorure d'hydrogène comprend ainsi 32 % en poids de fluorure d'hydrogène.

Le mélange réactionnel est alors amené à 55°C en plongeant le réacteur dans un bain thermostatisé, préchauffé. La pression est à ce stade de 5.10⁵ Pa. On introduit ensuite 9,6 g de chlore provenant d'un cylindre. La température passe à 65°C et est maintenue à cette valeur tandis que la pression autogène augmente en cours d'essai pour atteindre environ 7,5.10⁵ Pa après 15 minutes.

Des échantillons sont prélevés à l'aide d'un tube plongeant. Ils sont directement recueillis dans une ampoule contenant un mélange biphasique constitué d'une solution aqueuse de bicarbonate de soude saturée et de tétrachlorure de carbone. Après décantation et séchage sur CaCl₂, la phase organique est analysée par chromatographie en phase gazeuse.

Le tableau 1 reprend les teneurs (rapportées au 1,1-dichloro-1-fluoroéthane) en impuretés chlorées et chlorofluorées dans le mélange initial de 1,1-dichloro-1-fluoroéthane et de fluorure d'hydrogène puis 15 minutes après l'introduction du chlore.

**TABLEAU 1**

| | Concentration (mg/kg HFA-141b) | |
|---|---|---|
| | Mélange réactionnel initial | après traitement t = 15 min. |
| Chlorure de vinylidène | 2250 | < 3 |
| Dichloroacétylène | 2 | < 3 |
| 1,2-dichloroéthylène trans | 187 | < 3 |
| 1,2-dichloro-1-fluoroéthylène cis | 105 | < 3 |
| 1,2-dichloro-1-fluoroéthylène trans | 104 | < 3 |
| 1-chloro-1-fluoroéthylène | 6 | < 3 |
| 1-chloro-1,1-difluoroéthane | 12 | 2060 |

Après seulement 15 minutes de traitement, il ne reste dans le mélange que moins de 3 ppm de chacune des impuretés insaturées examinées, notamment de chlorure de vinylidène. On note également la formation de 1-chloro-1,1-difluoroéthane, facilement séparable du 1,1-dichloro-1-fluoroéthane par distillation.

On peut alors aisément obtenir par distillation du 1,1-dichloro-1-fluoroéthane de grande pureté.

### Exemple 2 R

Dans un autoclave en alliage HASTELLOY de 0,5 litre, équipé d'un agitateur, préalablement refroidi à -35°C et mis sous vide de 1500 Pa, on introduit par aspiration 380 g de 1,1-dichloro-1-fluoroéthane à épurer contenant 3,2 ppm de FeCl₃. On n'y ajoute pas de fluorure d'hydrogène.

Le mélange réactionnel est alors amené à 58°C en plongeant le réacteur dans un bain thermostatisé, préchauffé. La pression est à ce stade de 2,2.10⁵ Pa. On introduit ensuite 16 g de chlore provenant d'un cylindre. La température passe à 65°C et est maintenue à cette valeur tandis que la pression autogène augmente en cours d'essai pour atteindre environ 3,8.10⁵ Pa après 15 minutes.

Des échantillons sont prélevés à l'aide d'un tube plongeant. Ils sont directement recueillis dans une ampoule contenant une solution aqueuse de bicarbonate de soude saturée. Après décantation et séchage sur CaCl₂, la phase organique est analysée par chromatographie en phase gazeuse.

Le tableau 2 reprend les teneurs (rapportées au 1,1-dichloro-1-fluoroéthane) en impuretés chlorées et chlorofluorées dans le mélange initial de 1,1-dichloro-1-fluoroéthane puis 15 minutes après l'introduction du chlore.

**TABLEAU 2**

| | Concentration (mg/kg HFA-141b) | |
|---|---|---|
| | Mélange réactionnel initial | après traitement t = 15 min. |
| Chlorure de vinylidène | 2200 | 660 |
| Dichloroacétylène | 3 | 1 |
| 1,2-dichloroéthylène trans | 190 | 77 |
| 1,2-dichloro-1-fluoroéthylène cis | 106 | 51 |
| 1,2-dichloro-1-fluoroéthylène trans | 112 | 64 |
| 1-chloro-1-fluoroéthylène | 5 | 2 |
| 1-chloro-1,1-difluoroéthane | 13 | 7 |

### Exemple 3 R

Dans un autoclave en alliage HASTELLOY de 0,5 litre, équipé d'un agitateur, préalablement refroidi à -35°C et mis sous vide de 1500 Pa, on introduit par aspiration 381 g de 1,1-dichloro-1-fluoroéthane à épurer contenant 4,6 ppm de FeCl₃. On y ajoute 2 g de fluorure d'hydrogène, préalablement maintenu à température ambiante dans un cylindre en acier inoxydable. Le mélange de 1,1-dichloro-1-fluoroéthane brut et de fluorure d'hydrogène comprend ainsi 0,5 % en poids de fluorure d'hydrogène.

Le mélange réactionnel est alors amené à 60°C en plongeant le réacteur dans un bain thermostatisé, préchauffé. La pression est à ce stade de 3,2.10⁵ Pa. On introduit ensuite 22,9 g de chlore provenant d'un cylindre. La température passe à 65°C et est maintenue à cette valeur tandis que la pression autogène augmente en cours d'essai pour atteindre environ 4,5.10⁵ Pa après 15 minutes.

Des échantillons sont prélevés à l'aide d'un tube plongeant. Ils sont directement recueillis dans une ampoule contenant un mélange biphasique constitué d'une solution aqueuse de bicarbonate de soude saturée et de tétrachlorure de carbone. Après décantation et séchage sur CaCl₂, la phase organique est analysée par chromatographie en phase gazeuse.

Le tableau 3 reprend les teneurs (rapportées au 1,1-dichloro-1-fluoroéthane) en impuretés chlorées et chlorofluorées dans le mélange initial de 1,1-dichloro-1-fluoroéthane et de fluorure d'hydrogène puis 15 minutes après l'introduction du chlore.

**TABLEAU 3**

| | Concentration (mg/kg HFA-141b) | |
|---|---|---|
| | Mélange réactionnel initial | après traitement t = 15 min. |
| Chlorure de vinylidène | 1965 | 935 |
| Dichloroacétylène | 14 | < 3 |
| 1,2-dichloroéthylène trans | 158 | 109 |
| 1,2-dichloro-1-fluoroéthylène cis | 101 | 94 |
| 1,2-dichloro-1-fluoroéthylène trans | 122 | 94 |
| 1-chloro-1-fluoroéthylène | 7 | 3 |
| 1-chloro-1,1-difluoroéthane | 14 | 12 |

La comparaison des résultats de l'exemple 1, selon l'invention, avec ceux des exemples 2 et 3, donnés à titre de référence, atteste, d'une part, de l'efficacité remarquable du procédé selon l'invention, et d'autre part des résultats surprenants auxquels il conduit, dans la mesure où l'absence de fluorure d'hydrogène (exemple de référence 2) conduit à des résultats meilleurs que ceux obtenus en présence d'une quantité de fluorure d'hydrogène inférieure (exemple de référence 3) à celle utilisée dans le procédé selon l'invention.

## Revendications

1. Procédé pour l'épuration de 1,1-dichloro-1-fluoroéthane brut par traitement avec du chlore en présence d'acide de Lewis et de fluorure d'hydrogène puis distillation, caractérisé en ce que le fluorure d'hydrogène est présent lors du traitement avec du chlore à raison d'au moins 5 % en poids par rapport au mélange de 1,1-dichloro-1-fluoroéthane brut et de fluorure d'hydrogène.

2. Procédé selon la revendication 1, caractérisé en ce que le fluorure d'hydrogène est présent lors du traitement avec du chlore à raison d'au moins 15 % en poids par rapport au mélange de 1,1-dichloro-1-fluoroéthane brut et de fluorure d'hydrogène.

3. Procédé selon l'une quelconque des revendications 1 ou 2, caractérisé en ce que le fluorure d'hydrogène présent lors du traitement avec du chlore ne dépasse pas 50 % en poids par rapport au mélange de 1,1-dichloro-1-fluoroéthane brut et de fluorure d'hydrogène.

4. Procédé selon l'une quelconque des revendications précédentes, caractérisé en ce que le traitement avec du chlore est réalisé en présence de moins de 10 ppm d'acide de Lewis par rapport au 1,1-dichloro-1-fluoroéthane brut.

5. Procédé selon l'une quelconque des revendications précédentes, caractérisé en ce que le traitement avec du chlore est réalisé en présence d'au moins 0,15 ppm d'acide de Lewis par rapport au 1,1-dichloro-1-fluoroéthane brut.

6. Procédé selon l'une quelconque des revendications précédentes, caractérisé en ce que l'acide de Lewis est FeCl₃.

7. Procédé selon l'une quelconque des revendications précédentes, caractérisé en ce que le traitement avec du chlore est réalisé en présence de 0,1 à 12 % en poids de chlore par rapport au poids du 1,1-dichloro-1-fluoroéthane brut.

8. Procédé selon l'une quelconque des revendications précédentes, caractérisé en ce que le traitement avec du chlore est réalisé à une température de 0 à 90°C.

9. Procédé selon l'une quelconque des revendications précédentes, caractérisé en ce que le traitement avec du chlore ne dépasse pas 30 minutes.

## Claims

1. Process for the purification of crude 1,1-dichloro-1-fluoroethane by treatment with chlorine in the presence of Lewis acid and hydrogen fluoride and then distillation, characterised in that hydrogen fluoride is present during the treatment with chlorine at a ratio of at least 5 % by weight with respect to the mixture of crude 1,1-dichloro-1-fluoroethane and hydrogen fluoride.

2. Process according to Claim 1, characterised in that hydrogen fluoride is present during the treatment with chlorine at a ratio of at least 15 % by weight with respect to the mixture of crude 1,1-dichloro-1-fluoroethane and hydrogen fluoride.

3. Process according to either of Claims 1 or 2, characterised in that hydrogen fluoride present during the treatment with chlorine does not exceed 50 % by weight with respect to the mixture of crude 1,1-dichloro-1-fluoroethane and hydrogen fluoride.

4. Process according to any one of the above claims, characterised in that the treatment with chlorine is carried out in the presence of less than 10 ppm of Lewis acid with respect to crude 1,1-dichloro-1-fluoroethane.

5. Process according to any one of the above claims, characterised in that the treatment with chlorine is carried out in the presence of at least 0.15 ppm of Lewis acid with respect to crude 1,1-dichloro-1-fluoroethane.

6. Process according to any one of the above claims, characterised in that the Lewis acid is FeCl₃.

7. Process according to any one of the above claims, characterised in that the treatment with chlorine is carried out in the presence of 0.1 to 12 % by weight of chlorine with respect to the weight of crude 1,1-dichloro-1-fluoroethane.

8. Process according to any one of the above claims, characterised in that the treatment with chlorine is carried out at a temperature of 0 to 90°C.

9. Process according to any one of the above claims, characterised in that the treatment with chlorine does not exceed 30 minutes.

## Patentansprüche

1. Verfahren zur Reinigung von rohem 1,1-Dichlor-1-fluorethan durch Behandlung mit Chlor in Gegenwart einer Lewissäure und Fluorwasserstoff, anschließend Destillation, dadurch gekennzeichnet, daß der Fluorwasserstoff während der Behandlung mit Chlor in einer Menge von wenigstens 5 Gewichts-%, bezogen auf das Gemisch von rohem 1,1-Dichlor-1-fluorethan und Fluorwasserstoff, vorhanden ist.

2. Verfahren gemäß Anspruch 1, dadurch gekennzeichnet, daß der Fluorwasserstoff während der Behandlung mit Chlor in einer Menge von wenigstens 15 Gewichts-%, bezogen auf das Gemisch von rohem 1,1-Dichlor-1-fluorethan und Fluorwasserstoff, vorhanden ist.

3. Verfahren gemäß einem der Ansprüche 1 oder 2, dadurch gekennzeichnet, daß der während der Behandlung mit Chlor vorhandene Chlorwasserstoff 50 Gewichts-%, bezogen auf das Gemisch von rohem 1,1-Dichlor-1-fluorethan und Fluorwasserstoff, nicht übersteigt.

4. Verfahren gemäß einem der vorhergehenden Ansprüche, dadurch gekennzeichnet, daß die Behandlung mit Chlor in Gegenwart von weniger als 10 ppm der Lewissäure, bezogen auf das rohe 1,1-Dichlor-1-fluorethan, durchgeführt wird.

5. Verfahren gemäß einem der vorhergehenden Ansprüche, dadurch gekennzeichnet, daß die Behandlung mit Chlor in Gegenwart von wenigstens 0,15 ppm der Lewissäure, bezogen auf das rohe 1,1-Dichlor-1-fluorethan, durchgeführt wird.

6. Verfahren gemäß einem der vorhergehenden Ansprüche, dadurch gekennzeichnet, daß die Lewissäure FeCl₃ ist.

7. Verfahren gemäß einem der vorhergehenden Ansprüche, dadurch gekennzeichnet, daß die Behandlung mit Chlor in Gegenwart von 0,1 bis 12 Gewichts-% Chlor, bezogen auf das Gewicht des rohen 1,1-Dichlor-1-fluorethans, durchgeführt wird.

8. Verfahren gemäß einem der vorhergehenden Ansprüche, dadurch gekennzeichnet, daß die Behandlung mit Chlor bei einer Temperatur von 0 bis 90 °C durchgeführt wird.

9. Verfahren gemäß einem der vorhergehenden Ansprüche, dadurch gekennzeichnet, daß die Behandlung mit Chlor 30 Minuten nicht überschreitet.
